# EUROPEAN PATENT APPLICATION

(11) **EP 1 366 761 A1**
(43) Date of publication of application: **03.12.2003**
(21) Application number: 02712309.0
(22) Date of filing: 13.02.2002
(51) Int. Cl.: A61K 9/20, A61K 47/36, A61K 47/42, A61K 47/32, A61K 47/38

(54) **GEL PREPARATIONS FOR INTERNAL USE**

(30) Priority: 13.02.2001 JP 2001035074
(71) Applicant: TAISHO PHARMACEUTICAL CO. LTD, Tokyo 171 (JP)
(72) Inventor: NAKAMURA, Tohru, TAISHO PHARMACEUTICAL CO.,LTD, Toshima-ku, Tokyo 170-8633 (JP)
(74) Representative: HOFFMANN - EITLE
(86) International application number: JP0201181
(87) International publication number: WO02064120

(57) **Abstract**

There is disclosed a gel preparation for oral administration which contains a first eatable gel containing a medicinally effective ingredient and having a resolvability in the digestive tract, and a second eatable gel containing a medicinally effective ingredient and showing a behavior in the digestive tract different from that of the first eatable gel, the second eatable gel being contained in the first eatable gel.

The present invention provides a gel preparation for oral administration suitable for drugs or quasi-drugs which can be easily taken and can control the release time and release rate of the medicinally effective ingredient.

## Description

### TECHNICAL FIELD

The present invention relates to gel preparations for administration. More specifically, it relates to gel preparations for oral administration suitable for drugs or quasi-drugs which can be easily taken and can control the release time and release rate of the medicinally effective ingredient.

### BACKGROUND ART

There has been known that, in some cases, oral administration of a medicinal ingredient causes rapid rise of the blood concentration of the medicinal ingredient at first, followed by decrease of the blood concentration thereof with the passage of time.

However, it has also been known that the blood concentration of which the medicinally effective ingredient shows the drug action lies in a certain constant range called a therapeutic range (higher than the concentration wherein the drug action appears and lower than the concentration wherein the toxicity appears), and thereby, when the concentration of the medicinally effective ingredient is higher than this range, the toxicity appears, and it becomes rather harmful. On the other hand, however, suppression of the highest concentration within the range of the therapeutic area in order to avoid this toxicity leads to the loss of drug action in a short time, and therefore requires frequent administration of the medicine.

As a method of keeping the blood concentration of the medicinally effective ingredient long within the therapeutic range without suffering from toxicity, there is known a method comprising releasing a constant amount of the effective ingredient fast to rapidly make the blood concentration of the ingredient higher than the minimum effective concentration of drug, and thereafter, releasing the remaining effective ingredient at a comparatively slow rate in view of the metabolic rate thereof.

In order to achieve the above-mentioned method, the release pattern of the effective ingredients required for the preparations is a two phase release pattern containing a fast initial release (fast release part) and a subsequent slow release (slow release part), and it is necessary to control the content ratio of the effective ingredients distributed to the fast release part and the slow release part as well as to control the release rate of the slow release part in order to suit the drug action appearance concentration and the metabolic rate of the active ingredient used.

As such a preparation to control the release of the effective ingredient, there has recently been widely used capsule preparations filled with a granule (including the active ingredient) previously covered with a film to control the release.

However, this capsule preparation has a small dynamic deformability because of solid dosage form and a high cohesiveness to the oral cavity and the esophageal mucous membrane. Accordingly, it cannot be said that this preparation is easily taken, especially for the elderly person or infant with low swallowing capability. In addition, there is a problem that large size capsule preparations cannot be taken easily for the adult with a developed swallowing capability.

The present invention has been completed in view of the above-mentioned situation. The problem underlying the present invention is to provide a preparation which can properly control the release of the medicine while having an excellent oral administration properties.

### DISCLOSURE OF THE INVENTION

As a result of continued studies in order to solve the above-mentioned problem, the present inventors have paid attention to gel preparations (e.g., JP 10-236983 A and JP 2000-256216 A) which had been actively applied as an easy-to-take preparation in recent years, and have found that the above-mentioned problem can be solved by combining two or more of these preparations. In more detail, the present invention has been accomplished based on the finding that combined use of two gels having a different digestibility, which gels are known to have a capacity of controlling the release rate at some degree but have a simple release pattern and are not expected to have release control property including the control of release of the above-mentioned two phase release, permits proper control of the release and absorption of the medicinally effective ingredients in vivo while keeping good administration ability.

Thus, the present invention is to provide a gel preparation for oral administration which comprises a first eatable gel containing a medicinally effective ingredient and having a resolvability in the digestive tract, and a second eatable gel containing a medicinally effective ingredient and showing a behavior in the digestive tract different from that of the first eatable gel, the second eatable gel being contained in the first eatable gel.

### BRIEF DESCRIPTION OF THE DRAWINGS

Fig. 1 is a figure that shows the results of the release behavior of the effective ingredient by Experiment 1 concerning the fast release gels 1 to 5.
Fig. 2 is a figure that shows the results of the release behavior of the effective ingredient by Experiment 2 concerning the slow release gels 1 to 4.
Fig. 3 is a figure that shows the results of the release behavior of the effective ingredient by Experiment 3 concerning the gel preparations of products 1 to 3 according to the present invention and of comparative product 1.

### BEST MODE FOR CARRYING OUT THE INVENTION

The gel preparation for oral administration according to the present invention can be prepared by using two different eatable gels. Preferably, these gels do not have mutual solibility substantially.

The difference of the two eatable gels used in the present invention is in the resolvability in the digestive tract. Thus, for example, when the first eatable gel is resolvable in the stomach, the second eatable gel is not resolvable in the stomach and resolvable in the intestinal tract, or alternatively, the second eatable gel is resolvable neither in the stomach nor in the intestinal tract. In the present specification, "resolvability" is a concept that encompasses disintegration and dissolution as well as general degradation. In addition, "behavior" refers to the performances that eatable gels possess (e.g., resolvability, drug release, etc.).

The first eatable gel according to the present invention includes a gel which is soft and not mucoadhesive, and easily degradable, disintegrative and soluble in neutral to acidic range. In contrast, the second eatable gel according to the present invention includes a gel which does not have mutual solubility with the first eatable gel, and is neither degradable, nor disintegrative, nor soluble in acidic range.

Specific examples of the above-mentioned first eatable gel are not particularly limited so far as they are eatable and resolvable in neutral to acidic range, but the gels prepared by using a gelling agent such as carrageenan and gelatin are preferable, and the gels prepared by using kappa-carrageenan as a gelling agent are more preferable. When the first eatable gel is prepared by using carrageenan as a gelling agent, preferable amount of the carrageenan is preferably used in an approximate amount of from 0.3 to 3.0% by weight (hereinafter referred to as %) based on the whole weight of the gels, and when gelatin is used, it is preferably used in an approximate amount of from 1 to 10% based on the whole weight of the gels.

On the other hand, specific examples of the second eatable gel are not particularly limited so far as they are eatable and not resolvable in acidic range. Preferable are gels that are prepared by using agar, gelan gum, an alginic acid salt, carboxymethylcellulose calcium, pectin, polyvinyl alcohol, a polyacrylic acid salt, xanthan gum, locust bean gum, etc., as a gelling agent. These gelling agents can be used alone or in combination of two or more.

Among the above-mentioned second eatable gels, more preferable are gels which are prepared by using an alginic acid salt especially such as sodium alginate as a gelling agent. When an alginic acid salt is used as a gelling agent, the release rate (slow release) of the medicinally effective ingredient can be controlled by adjusting the molar ratio (M/G ratio) of mannuronic acid to guluronic acid, components of alginic acid. In general, the release rate of the medicinally effective ingredient can be slowed down by enlarging the M/G ratio (by enlarging the ratio of mannuronic acid). The M/G ratio where an alginic acid salt is used as a gelling agent ranges preferably from 0.3 to 6.0, and more preferably from 0.5 to 3.0. The second eatable gel is preferably used in an approximate amount of 10 to 70% based on the whole weight of the gels, and in case where the second eatable gel is prepared by using an alginic acid salt as a gelling agent, the alginic acid salt is preferably used in an approximate amount of from 0.5 to 20% based on the whole weight of the gels.

For the gel preparation for oral administration according to the present invention, a group of two or more gel particles showing different behavior from each other in the digestive tract can be also used as the second eatable gel. That is, a portion of the second gel may be constituted with gels that can be resolved in the small intestine upper region, and the other portion of the second gel may be constituted with either gels that can be resolved in the small intestine inferior region or gels that cannot be resolved in the alimentary canal (even though the gels are not resolved by the alimentary canal, the medicinally effective ingredient is gradually released from the gels).

For the gel preparation for oral administration according to the present invention, the first and second gels which are both resoluble in the intestinal tract but resoluble in different regions of the intestinal tract, respectively, can be used as the first and second eatable gels. For example, a gel which can be resolved in the small intestine upper region can be used as the first eatable gel, and another gel which can be resolved in the small intestine inferior region can be used as the second eatable gel.

For the production of the gel preparation for oral administration according to the present invention, medicinally effective ingredients are incorporated into the first eatable gel and the second eatable gel. The medicinally effective ingredients incorporated therein may be one or more.

The medicinally effective ingredients contained in the gel preparation for oral administration according to the present invention are, but not limited to, any medicinally effective ingredients that can be usually used for orally administered drugs such as, for example, acetaminophen, ibuprofen, loxoprofen and salts thereof, bromhexine hydrochloride, guaifenesin, guaiacolsulfonic acid salts and derivatives thereof, lysozyme chloride, dihydrocodeine phosphate, noscapine, dextromethorphan and salts thereof, methylephedrine hydrochloride, carbinoxamine maleate, caffeine, ascorbic acid, diclofenac sodium, domperidone, famotidine, cimetidine, riboflavin tetrabutyrate, terfenadine, methoxyphenamine and salts thereof, vancomycin, ciclosporin, scopolamine and salts thereof, meclizine hydrochloride, chlorpheniramine maleate, ethenzamide, phenylpropanolamine hydrochloride, pseudoephedrine, phenylephrine hydrochloride, bromovalerylurea, belladonna total alkaloid, dicyclomine hydrochloride, bromelain, carbazochrome, ubidecarenon, hepronicate, isopropylantipyrine, clarithromycin, aspirin and salts thereof.

The properties of the gel preparations for oral administration according to the present invention can vary depending upon the number and type of the medicinally effective ingredients, or upon the content ratio of the first eatable gel and the second eatable gel, as well as upon the above-mentioned character of the gels.

For example, in case where the first eatable gel is resolvable in acidic condition, and the second eatable gel is not resolvable in acidic condition, and where the same single medicinally effective ingredient is used, the medicinally effective ingredient in the first eatable gel is released in the stomach, and the medicinally effective ingredient in the second eatable gel is gradually released in the intestinal tract, and therefore, a fast release and a slow release required for the two phase release pattern can be satisfied. In addition, the blood concentration of the medicinally effective ingredient can be maintained to the therapeutic range for a long time by adjusting the content ratio of the first eatable gel of the fast release part to the second eatable gel of the slow release part, or adjusting the amount of the medical effective ingredient contained.

In the gel preparation for oral administration according to the present invention, it is possible to contain, besides the above-mentioned medicinally effective ingredients, any components that can be usually contained for drugs and the quasi-drugs in the first and second gel preparations, as long as the effects of the present invention are not adversely affected.

These components include dispersion media, sweeteners, stabilizers, antiseptic agents, pH regulators, emulsifying agents, solublizing agents, coloring agents and the flavoring agents, etc.

Among them, the dispersion media include those which are usually used for drugs and quasi-drugs such as, for example, water, alcohol, propylene glycol, glycerin, and a mixture thereof.

The sweeteners include those which are usually used for drugs and quasi-drugs such as, for example, sugar, D-sorbitol, xylitol, D-mannitol, maltitol, stevioside and sodium saccharide.

The stabilizers include those which are usually used for drugs and quasi-drugs such as, for example, EDTA-2Na, BHT, sodium sulfite, erythorbic acid and propyl gallate.

The antiseptic agents include those which are usually used for drugs and quasi-drugs such as, for example, benzoic acid and salts thereof, sorbic acid and salts thereof, parabens and dehydroacetic acid. The pH regulators include those which are usually used for drugs and quasi-drugs such as, for example, citric acid and salts thereof, tartaric acid and salts thereof, hydrochloric acid, sodium hydroxide, ammonia water, sodium carbonate, lactic acid and salts thereof, phosphoric acid and salts thereof, malic acid and salts thereof, and glycine.

Furthermore, the emulsifying agents include those which are usually used for drugs and quasi-drugs such as, for example, polyoxyethylene sorbitan fatty acid ester, sucrose ester of fatty acid, polyoxyethylene fatty acid ester, polyglycerin fatty acid ester, polyoxyethylene polyoxypropylene block polymer and sorbitan fatty acid ester; the solublizing agents include those which are usually used for drugs and quasi-drugs such as, for example, polyethylene glycol, food oils and fatty acid triglycerides; and the flavoring agents include those which are usually used for drugs and quasi-drugs such as, for example, menthols, various flavors and essential oils.

For the production of the gel preparation for oral administration according to the present invention by using the above components, for example, the second eatable gel is produced according to a known gel production method, the produced gel is properly adjusted in number and size, the adjusted gel is dispersed to a solution of the first eatable gel, and the first eatable gel is solidified with cooling.

Alternatively, in case where the gelling agent of the second eatable gel is LM-pectin or alginic acid, the gel preparation for oral administration according to the present invention can be produced by adding drops containing a medicinally effective ingredient and a gelling agent of the second eatable gel and adjusted to a desired size to a melted solution of the first eatable gel containing calcium ion, and solidifying the resulting mixture with cooling for the preparation.

The latter method is explained in more detail as follows with reference to an example using kappa-carrageenan as a gelling agent of the first eatable gel and using sodium alginate as a gelling agent of the second eatable gel.

Thus, a warming solution (the first gel solution) containing kappa-carrageenan, calcium lactate and a medicinally effective ingredient is prepared in a given molding container. Then, a solution (the second gel solution) containing sodium alginate and a medicinally effective ingredient is prepared. The prepared solution is added dropwise to the first gel solution. The sodium alginate in the second gel solution dropwise added is reacted with the calcium ion in the first gel solution. By the reaction of the resulting calcium alginate, the second gel solution is gelated in the first gel solution to form globular or granular gels (the second eatable gel). Then, after the given number of globular or granular gels are formed, the first gel solution is cooled to gelate kappa-carrageenan. The formed gels (the first eatable gel) are taken out of the molding container to obtain a gel preparation for oral administration according to the present invention.

According to an alternative method, a separately prepared second gel is added to and dispersed in a certain amount of a first gel solution, and filled the resulting gel into a given container and cooled, thereby the first eatable gel is formed and taken out.

In addition, there is also utilized a gelation method using the difference of the gelation temperatures of the gelling agents, which comprises forming the second eatable gel in the first gel solution, lowering the temperature, and gelating the first gel solution.

The thus-obtained gel preparation for oral administration according to the present invention permits an ideal drug release through variable adjustment of the release pattern of the medicinally effective ingredient contained therein.

That is, the two phase release pattern can be obtained by incorporating separately the same single medicinally effective ingredient into the first eatable gel (fast release part) and into the second eatable gel (slow release part). The release rate in vivo can be controlled by adjusting the content ratio of the medicinally effective ingredients in the fast release part and in the slow release part or selecting the gelling agents used. Therefore, the blood concentration of the medicinally effective ingredient can be made optimum.

Furthermore, the gel preparation for oral administration according to the present invention is characterized by its easy-to-take property attributable to the use of gels which are eatable, soft and not mucoadhesive, even though they contain a medicinally effective ingredient having bitterness, etc.

### Examples

The present invention is illustrated in more detail with reference to the following examples; however, the invention is in no way limited thereto.

### Production Example 1

### Production of Fast Release Part Gel (1)

1 Gram of acetaminophen, 0.5 g of gelan gum, 0.2 g of calcium lactate and 25 g of sugar were accurately weighed, and mixed in powder state. To the resulting mixture was added 73.3 g of purified water, followed by dispersion with stirring.

Then, the dispersed solution was heated to 80°C, the dispersing solid components were dissolved, and each 2 g of the solution was filled into a cylindrical container (13 mm in diameter, 20 mm in depth, and made of polypropylene), and solidified with cooling at 20 - 25°C to give fast release part gel 1.

### Production Example 2

### Production of Fast Release Part Gel (2)

The same formulation and production method as in Production Example 1 was repeated except for replacing 0.5 g of gelan gum with 0.5 g of kappa-carrageenan, and replacing 0.2 g of calcium lactate with the same amount of purified water, to obtain fast release part gel 2.

### Production Example 3

### Production of Fast Release Part Gel (3)

The same formulation and production method as in Production Example 1 was repeated except for replacing 0.5 g of gelan gum with 0.5 g of iotacarrageenan, and replacing 0.2 g of calcium lactate with the same amount of purified water, to obtain fast release part gel 3.

### Production Example 4

### Production of Fast Release Part Gel (4)

The same formulation and production method as in Production Example 1 was repeated except for replacing 0.5 g of gelan gum with 0.5 g of agar (Japanese Pharmacopoeia), and replacing 0.2 g of calcium lactate with the same amount of purified water, to obtain fast release part gel 4.

### Production Example 5

### Production of Fast Release Part Gel (5)

1 Gram of acetaminophen, 1 g of LM pectin, 1 g of calcium lactate and 25 g of sugar were accurately weighed, and these were mixed in powder state. To the resulting mixture was added 72 g of purified water, followed by dispersion with stirring.

Then, the dispersed solution was heated to 80°C, the dispersing solid components were dissolved, and each 2 g of the solution was filled into a container of the same specification as used in Production Example 1, and solidified with cooling at 20 - 25°C to give fast release part gel 5.

### Production Example 6

### Production of Fast Release Part Gel (6)

1.0 Gram of acetaminophen and 1.0 g of kappa-carrageenan were accurately weighed, and these were added to 98 g of purified water, and dispersed with stirring.

Then, the dispersed solution was heated to 80°C or higher, and the dispersing solid components were dissolved, and filled into a container of the same specification as used in Production Example 1, and solidified with cooling at 20 - 25°C to give fast release part gel 6.

### Production Example 7

### Production of Fast Release Part Gel (7)

The same formulation and production method as in Production Example 6 was repeated except for changing the amount of kappa-carrageenan to 1.3 g, and changing the amount of purified water to 97.7 g, to obtain fast release part gel 7.

### Production Example 8

### Production of Fast Release Part Gel (8)

The same formulation and production method as in Production Example 6 was repeated except for changing the amount of kappa-carrageenan to 2 g, and replacing 98 g of purified water with 97 g of 0.5 M phosphate buffer solution (pH 6.8), to obtain fast release part gel 8.

### Experiment 1

### Release Behavior of Effective Ingredient of Fast Release Part Gel

The release behaviors of the effective ingredient (acetaminophen) of fast release part gels 1 - 5 were verified by using the following conditions according to the dissolution test (Puddle Method) described in Japanese Pharmacopoeia. The quantitative analysis of the effective ingredient was carried out by HPLC method.

| (Test Conditions) | |
|---|---|
| Puddle revolution number | 50 rpm |
| dissolution medium | First solution of Japanese Pharmacopoeia (pH:1.2) |
| Temperature | 37°C |

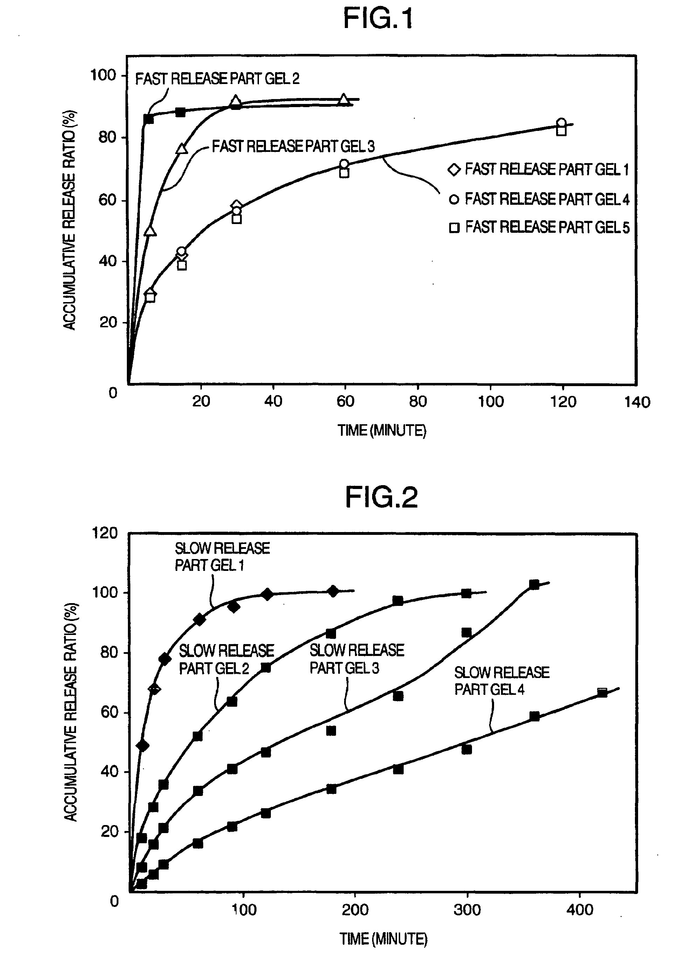

Fig. 1 shows the release behavior (relation between the elapsed time and the accumulative release ratio) of acetaminophen of fast release part gels in Experiment 1.

As apparent from the results, fast release part gels 2 and 3 containing carrageenan behave so as to release the medicinally effective ingredient in shorter time than the others. Therefore, it is verified that carrageenan is preferable, and kappa-carrageenan is especially preferable as the gelling agent for the fast release part.

### Production Example 9

### Production of Slow Release Part Gel (1)

1 Gram of acetaminophen and 2 g of sodium alginate [molar ratio of mannuronic acid to guluronic acid (hereinafter referred to as M/G ratio) is 0.6] were added to 97 g of purified water and dissolved.

Then, this solution was added dropwise to a 0.2 M calcium chloride aqueous solution containing 1% of acetaminophen by using an injector needle (1.0 mm in inside diameter). After the dropwise addition, the resulting solution was ripened one whole day and night to give gel beads.

The resulting gel beads were soaked for 10 minutes in 0.5 M phosphate buffer solution (pH 6.8) containing 1% of acetaminophen for treatment with phosphoric acid salt. After the treatment, the gel was ripened one whole day and night to give slow release part gel 1.

### Production Example 10

### Production of Slow Release Part Gel (2)

The same formulation and production method as in Production Example 9 was repeated except for changing the amount of sodium alginate.to 4 g, and changing the amount of purified water to 95 g, to obtain slow release part gel 2.

### Production Example 11

### Production of Slow Release Part Gel (3)

The same formulation and production method as in Production Example 9 was repeated except for using 4 g of sodium alginate of which the M/G ratio was 1.3, and changing the amount of purified water to 95 g, to obtain slow release part gel 3.

### Production Example 12

### Production of Slow Release Part Gel (4)

The same formulation and production method as in Production Example 9 was repeated except for using 4 g of sodium alginate of which the M/G ratio was 2.2, and changing the amount of purified water to 95 g, to obtain slow release part gel 4.

### Production Example 13

### Production of Slow Release Part Gel (5)

The same formulation and production method as in Production Example 9 was repeated except for using 3 g of sodium alginate of which the M/G ratio was 1.3, and changing the amount of purified water to 96 g, to obtain slow release part gel 5.

### Experiment 2

### Release Behavior of Effective Ingredient of Slow Release Part Gel

The release behaviors of the effective ingredient (acetaminophen) of slow release part gels 1 - 4 were verified by using the following conditions according to the dissolution test (Puddle Method) described in Japanese Pharmacopoeia. The quantitative analysis of the effective ingredient was carried out by HPLC method.

| (Test Conditions) | |
|---|---|
| Puddle revolution number | 50 rpm |
| dissolution medium | Second solution of Japanese Pharmacopoeia (pH:6.8) |
| Temperature | 37°C |

Fig. 2 shows the release behavior (relation between the elapsed time and the accumulative release ratio) of acetaminophen of slow release part gels in Experiment 2.

As apparent from the results, of the runs in which the content ratio of sodium alginate is changed, the gels containing higher content ratio of sodium alginate (slow release part gels 1 and 2) behave so as to release the medicinally effective ingredient more gently. Of the runs in which the content ratio of sodium alginate is kept constant, the gels tend to behave so as to release the medicinally effective ingredient more gently as the M/G ratio of sodium alginate rises (slow release part gels 2 - 4). Therefore, it is verified that the release rate of the medicinally effective ingredient can be controlled by changing the content ratio or M/G ratio of sodium carrageenan, the gelling agent of slow release part.

### Example 1

### Production of Gel Preparation for Oral Administration (1)

1.3 Grams (65% based on the whole gel preparation, hereinafter used by the same meaning) of fast release part gel 6 was weighed, and dissolved with heating at 60°C or higher.

0.7 Gram (35%) of slow release part gel 4 was added to the dissolved fast release part gel. The resulting mixture was filled into a container of the same specification as used in Production Example 1, cooled at 20 - 25°C, and the whole was gelated to give a gel preparation for oral administration (inventive product 1).

### Example 2

### Production of Gel Preparation for Oral Administration (2)

1.08 Grams (54%) of fast release part gel 7 was weighed, and dissolved with heating at 60°C or higher.

0.92 Gram (46%) of slow release part gel 5 was added to the dissolved fast release part gel. The resulting mixture was filled into a container of the same specification as used in Production Example 1, cooled at 20 - 25°C, and the whole was gelated to give a gel preparation for oral administration (inventive product 2).

### Example 3

### Production of Gel Preparation for Oral Administration (3)

1.14 Grams (57%) of fast release part gel 8 was weighed, and dissolved with heating at 60°C or higher.

0.86 Gram (43%) of slow release part gel 4 was added to the dissolved fast release part gel. The resulting mixture was filled into a container of the same specification as used in Production Example 1, cooled at 20 - 25°C, and the whole was gelated to give a gel preparation for oral administration (inventive product 3).

### Experiment 3

### Release Behavior of Effective Ingredient of Gel Preparation for Oral Administration

The release behavior of the effective ingredient (acetaminophen) of inventive products 1 - 3 and comparative product 1 composed only by fast release part gel 7 were verified by using the following conditions according to the dissolution test (Flow through cell method) described in Japanese Pharmacopoeia. As the dissolution medium, the first solution of Japanese Pharma-copoeia (pH 1.2 : 150 ml) was used from the start of the experiment to 30 minutes and, after that, the second solution of Japanese Pharmacopoeia (pH 6.8 : 300 - 900 ml) was used to the end.

Concerning the experiment conditions, the flow rate of the dissolution medium was 5.0 ml/minute, and the temperature was 37°C. The quantitative analysis of effective ingredient was carried out by HPLC method.

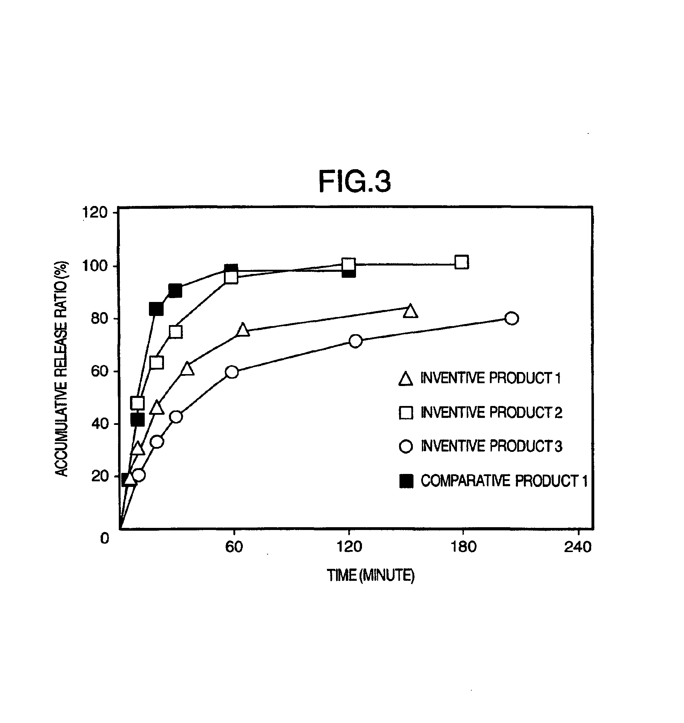

Fig. 3 shows the release behavior (relation between the elapsed time and the accumulative release ratio) of acetaminophen from the gel preparation to the dissolution medium in the flow through cell method. As apparent from the results, comparative product 1, composed only by fast release part gel, promptly releases acetaminophen immediately after the start of the experiment, and releases it gently afterwards. In contrast, inventive products 1 - 3, which are each complex gel of fast release part gel and slow release part gel, shows that these products release acetaminophen more gently than comparative product 1 after the start of the experiment.

It is verified that, combined use of fast release part gel and slow release part gel permits much more gentle, controlled release of medicinally effective ingredient as compared with fast release part gel alone.

Furthermore, it is verified from the results in Fig. 3 that each of the gel preparations of inventive products 1 - 3 has an original release behavior of its own, so that the release pattern of medicinally effective ingredient can be adjusted by changing the combination of fast release part gel and slow release part gel.

### Example 4

### Production of Gel Preparation for Oral Administration (4)

0.2 Gram of acetaminophen, 0.4 g of sodium alginate (M/G ratio: 2.2), 6 g of xylitol and 0.004 g of propylparaben were added to purified water so as to make the total weight 20 g, and dissolved with heating at 60 - 80°C to give preparation solution 1.

Separately, 0.2 g of kappa-carrageenan, 0.1 g of acetaminophen, 0.5 g of calcium lactate, 3.0 g of xylitol and 0.002 g of propylparaben were added to 6.698 g of purified water, sufficiently dispersed at room temperature, and dissolved with heating at 60-80°C to give preparation solution 2.

To preparation solution 2 was added dropwise, while keeping warm and gently stirring, the previously produced preparation solution 1 through an injector needle (1 mm in inside diameter). After the dropwise addition, the resulting solution was solidified with cooling at 5°C to give gel preparation for oral administration (inventive product 4).

### Example 5

### Production of Gel Preparation for Oral Administration (5)

0.1 Gram of acetaminophen, 0.2 g of sodium alginate (M/G ratio: 1.3), 0.2 g of low-methoxyl pectin, 2.8 g of xylitol and 0.002 g of propylparaben were added to 6.698 g of purified water, and dissolved with heating at 60 - 80°C to give preparation solution 1.

Separately, 0.2 g of kappa-carrageenan, 0.1 g of acetaminophen, 0.5 g of calcium lactate, 2.5 g of xylitol and 0.002 g of propylparaben were added to 6.698 g of purified water, sufficiently dispersed at room temperature, and dissolved with heating at 60-80°C to give preparation solution 2.

To the preparation solution 2 was added dropwise, while keeping warm and gently stirring, the previously produced preparation solution 1 through an injector needle (1 mm in inside diameter). After the dropwise addition, the resulting solution was solidified with cooling at 5°C to give gel preparation for oral administration (inventive product 5).

### Example 6

### Production of Gel Preparation for Oral Administration (6)

0.1 Gram of acetaminophen, 0.5 g of agar, 2.7 g of xylitol and 0.002 g of propylparaben were added to 6.698 g of purified water, sufficiently dispersed at room temperature, and dissolved with heating at 80-90°C. The resulting mixture was placed in a suitable container in the form of a sheet of 5 mm in thickness, and solidified with cooling at room temperature. The resulting gel was cut into 5 mm cubes to give gel 2.

Separately, 0.05 g of kappa-carrageenan, 0.1 g of acetaminophen, 3.15 g of xylitol and 0.002 g of propylparaben were added to 6.698 g of purified water, sufficiently dispersed at room temperature, and dissolved with heating at 50 - 70°C. To the solution was added, while keeping warm and gently stirring, the previously prepared gel 2, and the resulting solution was solidified with cooling at 5°C to give gel preparation for oral administration (inventive product 6).

The thus-obtained gel preparation for oral administration according to the present invention permits various adjustment of the release pattern of the medicinally effective ingredient contained therein and achieve an ideal drug release. For example, it can be advantageously used as a sustained release preparation, which maintains the blood concentration of therapeutic ingredient in the therapeutic range for a long term.

Furthermore, in this preparation, the medicinally effective ingredient is enclosed in the gel, so that the preparation reduce uncomfortable tastes such as bitterness. Also the preparation can easily be taken, because gels are inherently easily ingestible.

Accordingly, the gel preparation for oral administration according to the present invention can be widely used as a novel preparation having a wide functionality, and especially is the best as easy-to-take medicine for the elderly person or baby who feels difficulty in oral ingestion.

## Claims

1. A gel preparation for oral administration which comprises a first eatable gel containing a medicinally effective ingredient and having a resolvability in the digestive tract, and a second eatable gel containing a medicinally effective ingredient and showing a behavior in the digestive tract different from that of the first eatable gel, the second eatable gel being contained in the first eatable gel.

2. The gel preparation for oral administration according to Claim 1, wherein the second eatable gel is two or more gel particles dispersed in the first eatable gel.

3. The gel preparation for oral administration according to Claim 1, wherein the second eatable gel is a group of two or more gel particles, each of which is dispersed in the first eatable gel and shows a behavior different from each other in the digestive tract.

4. The gel preparation for oral administration according to Claim 1, wherein the second eatable gel is one gel particle.

5. The gel preparation for oral administration according to any one of Claims 1 to 4, wherein the first eatable gel is resolvable in the stomach, and the second gel is not resolvable in the stomach but resolvable in the intestinal tract.

6. The gel preparation for oral administration according to any one of Claims 1 to 4, wherein the first eatable gel is resolvable in the stomach, and the second gel is resolvable neither in the stomach nor in the intestinal tract.

7. The gel preparation for oral administration according to any one of Claims 1 to 4, wherein the first eatable gel is resolvable in a region of the intestinal tract, and the second eatable gel is resolvable in another region of the intestinal tract.

8. The gel preparation for oral administration according to any one of Claims 1 to 7, wherein the first eatable gel is a gel formed with carrageenan or gelatin.

9. The gel preparation for oral administration according to any one of Claims 1 to 4 and 6, wherein the second eatable gel is a gel formed with one or more gelling agents selected from the group consisting of agar, gelan gum, an alginic acid salt, carboxymethylcellulose calcium, pectin, polyvinyl alcohol, a polyacrylic acid salt, xanthan gum and locust bean gum.

10. The gel preparation for oral administration according to Claim 9, wherein the second eatable gel is formed with a gelling agent containing an alginic acid salt.

11. The gel preparation for oral administration according to Claim 10, wherein the alginic acid salt comprises 10 to 70% by weight of a whole weight of the gel.

12. The gel preparation for oral administration according to Claim 10 or 11, wherein the alginic acid salt contains mannuronic acid and guluronic acid in a mannuronic acid/guluronic acid (M/G) molar ratio of 0.3 to 6.0.

13. The gel preparation for oral administration according to Claim 1, wherein the medicinally effective ingredient in the first eatable gel is the same as the medicinally effective ingredient in the second eatable gel.
